# EUROPEAN PATENT SPECIFICATION

(11) **EP 0 478 664 B1**
(45) Date of publication and mention of the grant of the patent: **17.09.2003**
(21) Application number: 90909981.4
(22) Date of filing: 18.06.1990
(51) Int. Cl.: C12N 15/74, C12N 15/48, A61K 39/04, A61K 39/21, C12N 1/21

(54) **VECTOR-MEDIATED GENOMIC INSERTION AND EXPRESSION OF DNA IN BCG**
VERKTORVERMITTELTE GENOMINSERTION UND EXPRESSION VON DNA IN BCG
INSERTION GENOMIQUE A MEDIATION VECTORIELLE ET EXPRESSION D'ADN DANS LE BCG (BACILLE BILIE CALMETTE-GUERIN)

(30) Priority: 19.06.1989 US 367894
(43) Date of publication of application: 08.04.1992
(73) Proprietor: WHITEHEAD INSTITUTE FOR BIOMEDICAL RESEARCH, Cambridge, MA 02142 (US)
(72) Inventor: YOUNG, Richard, A., Winchester, MA 01890 (US); ALDOVINI, Anna, Winchester, MA 01890 (US)
(74) Representative: Price, Vincent Andrew
(86) International application number: US9003451
(87) International publication number: WO90015873

(56) References cited:
- WO-A-88/06626
- WO-A-90/00594
- PROCEEDINGS OF THE NATL. ACADEMY OF SCIENCES USA, vol. 85, September 1988, Washington, DC (US); S.B. SNAPPER et al., pp. 6987-6991
- JOURNAL OF BACTERIOLOGY, vol. 172, no. 2, February 1990, American Society for Microbiology; R.N. HUSSON et al., pp. 519-524

## Description

### Background

Mycobacteria represent major pathogens of man and animals. For example, tuberculosis is generally caused in humans by Mycobacterium (M.) tuberculosis and in cattle by Mycobacterium (M.) bovis (which can be transmitted to humans and other animals, in whom it causes tuberculosis). Tuberculosis remains widespread and is an important public health problem, particularly in developing countries. It is estimated that there are approximately 10 million cases of tuberculosis worldwide, with an annual mortality of 3 million. Joint International Union Against Tuberculosis and World Health Organization Study Group, Tubercle, 63:157-169 (1982).

Leprosy, which is caused by M. leprae, afflicts over 10 million people, primarily in developing countries. Bloom, B. R. and T. Godal, Review of Infectious Diseases, 5:657-679 (1984). M. tuberculosis and mycobacteria of the avium-intracellulare-scrofulaceum (MAIS) group represent major opportunistic pathogens of patients with acquired immunodeficiency disease (AIDS). Centers for Disease Control, Morbidity and Mortality Weekly Report, 34:774 (1986). M. pseudotuberculosis is a major pathogen of cattle.

On the other hand, Bacille Calmette-Guerin (BCG), an avirulent strain of M. bovis, is the most widely used human vaccine in the world and has been used as a live vaccine for more than 50 years. In the past 35 years, it has been administered to over 2.5 billion people, with remarkably few adverse effects (e. g., estimated mortality of 60/billion). BCG has been found in numerous studies to have protective efficacy against tuberculosis. However, it was found not to be effective in preventing pulmonary tuberculosis in some trials, for example in Southern India. Tuberculosis Prevention Trial, Madras, Indian Journal of Medical Research, 72 (suppl.):1-74 (1980).

Mycobacteria have been proposed for use as live vaccine vehicles. Genes encoding antigens from a variety of pathogens can be expressed in recombinant mycobacteria such that the adjuvant properties of the mycobacteria stimulate an immune response to the foreign antigens which, in turn, provide protective immunity against these pathogens. Mycobacteria offer several advantages as vaccine vehicles, including a long record of use in humans with a very low incidence of complications, stability in the field and single dose usage. The ability to manipulate the mycobacterial genome is essential to developing this candidate vaccine vehicle.

The ideal molecular genetic system includes a transformation method, vectors that can be manipulated in vitro, selectable nutritional and drug markers and an ability to replace specific genomic DNA sequences with exogenous sequences. A means by which exogenous DNA encoding a protein of interest can be introduced into the mycobacterial genome in place of specific genomic DNA sequences to produce a recombinant mycobacterium capable of expressing the encoded protein would be very useful.

WO90/00594 discloses recombinant mycobacteria having heterologous DNA encoding a lymphokine or immunopotentiator stably incorporated into the genomic DNA and controlled by the cl promoter.

WO/88/06626 also discloses recombinant mycobacteria expressing heterologous DNA but does not disclose the expression of DNA encoding immunopotentiators or lymphokines.

### Summary of the Invention

The present invention relates to the subject matter of the claims.

The expression cassette includes DNA of includes DNA of interest encoding the lymphokine or immunopatentiator and a regulated promoter region (which does not comprise the cl promoter); the two components are in such proximity to one another that expression of the DNA of interest in the recombinant mycobacterium is controlled by the regulated promoter region. The regulated promoter region is generally a mycobacterial promoter region, but can also be a regulated bacterial promoter, such as the E. coli lacZ promoter. For example, a heat shock protein promoter region or stress protein promoter region (e.g., hsp70, hsp60) can be inserted into a recombinant plasmid vector in close proximity to the DNA of interest, such that translation of the gene is controlled by the mycobacterial promoter region and ribosome binding sites.

The mycobacteria of the present invention may be produced by methods in which double homologous recombination occurs between plasmid-borne DNA sequences homologous to DNA sequences in the mycobacterial genome and the homologous mycobacterial sequences. As a result of the recombination event, a recombinant mycobacterium is produced which is very similar to the corresponding unmodified mycobacterium. Such recombinant mycobacteria include the expression cassette (i.e., DNA of interest and the regulated promoter region), which is not present in the unmodified mycobacterium. Such methods may be used to produce recombinant mycobacteria which do not contain vector DNA sequences or other sequences not normally present in mycobacteria other than those of the DNA of interest.

The resulting recombinant mycobacteria (e.g., recombinant BCG, recombinant M. smegmatis) are particularly useful as vehicles in which the DNA encoding the lymphokine or immunopotentiator can be expressed. Such vehicles can be used, for example, as regulatable vaccine vehicles which express a lymphokine or immunopotentiator.

The vaccine of the subject invention has important advantages over presently-available vaccines. In particular, the recombinant mycobacteria as defined in the claims may express the DNA encoding the lymphokine of immunopotentiator as a protein product without extraneous or additional proteins. This differs from presently available vaccine vehicles in which a gene or genes of interest are expressed in a recombinant form (e.g., fusion protein). Further, mycobacteria have adjuvant properties among the best currently known and, thus, stimulate a recipient's immune system to respond to other antigens with great effectiveness. This is a particularly valuable aspect of the vaccine because it induces cell-mediated immunity and will, thus, be especially useful in providing immunity against pathogens in cases where cell-mediated immunity appears to be critical for resistance. Second, the mycobacterium stimulates long-term memory or immunity. As a result, a single (one-time) inoculation can be used to produce long-term sensitization to protein antigens. Using the vaccine vehicle of the present invention, it is possible to prime long-lasting T cell memory, which stimulates secondary antibody responses neutralizing to the infectious agent or the toxin. This is useful, for example, against tetanus and diphtheria toxins, pertusis, malaria, influenza, herpes viruses and snake venoms.

BCG in particular has important advantages as a vaccine vehicle in that: 1) it is the only childhood vaccine currently given at birth; 2) in the past 40 years, it has had a very low incidence of adverse effects, when given as a vaccine against tuberculosis; and 3) it can be used repeatedly in an individual (e. g., in multiple forms).

A further advantage of BCG in particular, as well as mycobacteria in general, is the large size of its genome (approximately 3 x 10⁶ bp in length). Because the genome is large, it is able to accommodate a large amount of DNA from another source (i.e., DNA of interest) and, thus, can be used to make a multi-vaccine vehicle (i. e., one carrying DNA of interest encoding protective antigens for more than one pathogen).

### Brief Description of the Drawings

Figure 1 is a schematic representation of the construction of a marked target mycobacterial strain.
Figure 2 is a schematic representation of insertion of DNA of interest into a marked target BCG.
Figure 3 is a schematic representation of vector pY6014, which contains the mycobacterial PyrF gene and flanking DNA sequences.
Figure 4 is a schematic representation of vector pY6015, which contains the aph gene (encodes kanamycin resistence) inserted at the PyrF site of pY6014.
Figure 5 is a schematic representation of vector pY6016, which contains the mycobacterial PyrF gene and flanking DNA sequences of pY6014 with several restriction sites removed.
Figure 6 is a schematic representation of vector pY6017, which contains the hsp70 gene inserted ∼70bp downstream of PyrF in pY6016.
Figure 7 is a schematic representation of vector pY6018, which contains the hsp70 promoter and the HIV1 gag gene inserted in the hsp70 coding site of pY6017.
Figure 8 is a schematic representation of vector pY6019, which contains the hsp70 promoter and the HIV1 pol gene inserted in the hsp70 coding site of pY6017.
Figure 9 is a schematic representation of vector pY6020, which contains the hsp70 promoter and the HIV1 env gene inserted in the hsp70 coding site of pY6017.
Figure 10 is a schematic representation of vector pY6021, which contains the hsp70 promoter and the SIV1 gag gene inserted in the hsp70 coding site of pY6017.
Figure 11 is a schematic representation of vector pY6022, which contains the hsp70 promoter and the SIV1 pol gene inserted in the hsp70 coding site of pY6017.
Figure 12 is a schematic representation of vector pY6023, which contains the hsp70 promoter and the SIV1 env gene inserted in the hsp70 coding site of pY6017.

### Detailed Description of the Invention

Mycobacterium bovis-BCG (BCG or M. bovis-BCG) is an avirulent M. bovis derivative which is widely used throughout the world, particularly to provide protection against tuberculosis.

Because M. bovis-BCG has excellent adjuvant activity for induction of cell-mediated immunity, stimulates long-term memory (immunity) and has a low mortality associated with its use, it is an excellent candidate as a recombinant vehicle for the expression of proteins of interest, such as a protein antigen, enzymes, lymphokines, and immunopotentiators.

It is now possible to produce recombinant mycobacteria in which DNA encoding a lymphokine or immunopotentiator is expressed under the control of a regulated promoter region (which does not comprise the cI promoter). In particular, it is now possible to introduce such DNA in combination with the regulated promoter region, such as the heat shock protein (hsp)70 or the hsp60 promoter region. The resulting recombinant BCG has very similar characteristics to those of the unmodified BCG. The recombinant mycobacteria includes the lymphokine or immunopotentiator DNA and the (non-cI promoter) regulated promoter region, which are not in the unmodified BCG. Expression of the DNA of interest, to produce the encoded lymphokine or immunopotentiator is under the control of the regulated promoter region. The recombinant mycobacteria are useful as expression vehicles, in which expression of the lymphokine or immunopotentiator is under the control of the regulated promoter region and, thus, is regulatable.

The following is a description of construction of marked mycobacterial target strains; construction of integration vectors useful for introducing DNA of interest into marked mycobacterial target strains; production of recombinant mycobacteria having DNA of interest stably integrated into genomic DNA; and uses of the resulting recombinant mycobacteria. Although the following is described in terms of BCG, it is to be understood that the methods and vectors described can also be used to produce recombinant M. snegmatis, as well as other recombinant mycobacteria, such as: M. avium, M. phlei, M. fortuitum, M. lufu, M. paratuberculosis, M. habana, M. scrofulaceum, M. tuberculosis, and M. intracellulare.

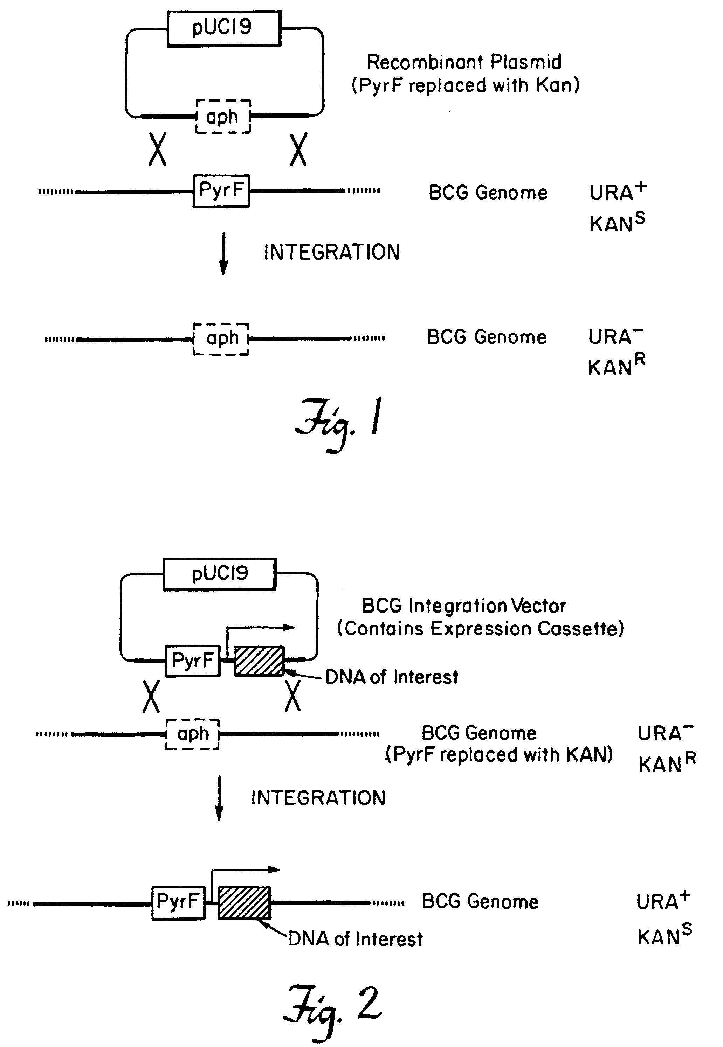

Figure 1 is a schematic representation of construction of a marked mycobacterial (BCG) target strain, which is the target for further manipulation using standard genetic engineering methods. A recombinant plasmid containing mycobacterial DNA in which the PyrF gene has been replaced with a gene encoding a selectable marker is used to transform M. bovis-BCG, using standard methods such as electroporation. A double homologous recombination event (indicated in Figure 1 by two Xs) occurs between plasmid-borne sequences homologous to sequences within the mycobacterial genome. For double homologous recombination to occur, plasmid-borne sequences homologous to sequences in the mycobacterial genome are positioned on either side of the gene encoding the selectable marker. The result is a replacement in the mycobacterial genome of the PyrF gene with the gene encoding the selectable marker (e.g., aph as represented in Figure 1). Transformants (i.e., mycobacteria in which the PyrF gene has been replaced by the selectable marker-encoding gene) exhibit a change in phenotype different from the phenotype observed in the unmodified mycobacterium. Transformants are selected on the basis of either of these characteristics.

The resulting marked mycobacterial target strain is used as a target for further manipulation, using known techniques, by which DNA of interest is integrated into mycobacterial genomic DNA. An integration vector such as that represented in Figure 2 is used for this purpose. The integration vector includes DNA sequences homologous to DNA sequences in the target mycobacterial genome, DNA encoding a selectable marker of mycobacterial origin, DNA encoding a protein or polypeptide of interest (DNA of interest) and DNA encoding a regulated mycobacterial promoter region. The remaining sequences present in the integration vector are those of an appropriate plasmid vector (such as pUC19). Construction of integration vectors of the present invention are described in detail below. The integration vector is introduced into the marked mycobacterial target strain using standard techniques (e.g., electroporation). A double homologous recombination event (indicated in Figure 2 by 2 Xs) occurs between plasmid-borne mycobacterial sequences which are homologous to sequences in the target mycobacterial genome and which flank the DNA encoding a selectable marker of mycobacterial origin (on one side) and the DNA of interest (on the other side). The result is a replacement in the marked target strain of the gene encoding a selectable marker (in Figure 2, aph) and flanking sequences with DNA homologous to mycobacterial sequences, the DNA encoding a selectable marker of mycobacterial origin, the DNA of interest, and DNA encoding a regulated mycobacterial promoter region. This is represented schematically in Figure 2. As shown, the double homologous recombination event results in recombinant mycobacteria which have two phenotypic characteristics useful for identifying and selecting cells containing the DNA of interest and regulated promoter region.

The recombinant BCG may differ from the unmodified BCG only by the presence of the DNA of interest and the regulated promoter region, as shown in Figure 2. Here, the mycobacterial genomic DNA has been changed (by replacement of the PyrF gene and flanking sequences with the aph gene and flanking sequences) and then "reconstructed" in such a manner that equivalents of the PyrF gene and flanking sequences are integrated, along with the expression cassette. The "net" change is incorporation into the mycobacterial genome of DNA of interest and a regulated promoter region.

The regulated promoter region present in the expression cassette can be any regulated promoter region functional in mycobacteria, with the prouiso that the promoter region does not comprise the cI promoter. The regulated promoter region includes the transcriptional promoter and translational start site, including the ribosome binding sites. In general, the promoter region is of mycobacterial origin. However, regulated bacterial promoters, such as the E. coli lacZ promoter, can also be used. In one embodiment of the present invention, the regulated promoter region is a heat shock promoter region (hsp) of mycobacterial origin, such as the BCG hsp70 or hsp60. Use of such a regulated promoter region provides an important advantage to the recombinant mycobacteria; particularly in expression vehicles which are vaccine vehicles. In circumstances, such as increased temperature or bacterial infection, in which an individual is stressed, the hsp promoters are turned up and enhanced protein production occurs. In the present invention, this is particularly useful because expression of the DNA of interest is under the control of the regulated promoter region, which is turned up and, thus, drives expression of the DNA of interest at a higher than normal level.

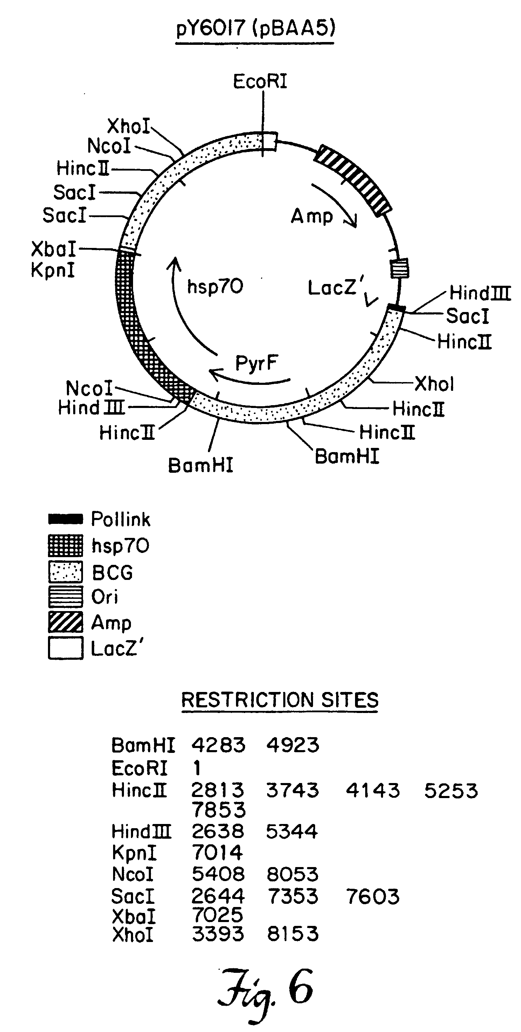

Until the present time, it has not been possible to produce recombinant nycobacterial expression vehicles in which DNA encoding a polypeptide or protein, such as one against which an immune response is desired, is stably integrated, at selected sites and in selected orientations, in genomic DNA. A mycobacterial promoter and ribosome binding site, such as a heat shook protein promoter region (e.g., hsp70, hsp60), may serve as regulatable expression signals controlling expression of the DNA encoding the lymphokine or immunopotentiator. As represented in Figure 6, the expression cassette can include a polylinker in sequences surrounding the mycobacterial sequences necessary for double homologous recombination (i.e., the PyrF gene). As a result, DNA of interest can be inserted into the mycobacterial genome and mycobacterial expression signals will control the level of production of the protein or polypeptide of interest. Selection of mycobacterial cells in which the PyrF-expression cassette-DNA of interest combination are stably integrated can be carried out as described previously.

The DNA encoding the lymphokine or immunopotentiator can be obtained by isolation of the naturally-occurring DNA; by cloning and amplification of the DNA sequence of interest, using known genetic engineering techniques (See, for example, Maniatis, T. et. al. Molecular Cloning: A Laboratory Manual, Cold Spring Harbor, N.Y. (1982).); or by mechanical or chemical synthesis (e.g., polymerase chain reaction (PCR)).

The vectors described above and in the following sections can be used to genetically engineer mycobacteria with DNA encoding a lymphokine or immunopotentiator.

As a result, it is possible to produce recombinant mycobacterial vaccines.

An important advantage of the method described herein, which is illustrated in Figure 2, is that integration of the DNA of interest occurs without concomitant integration of plasmid DNA into the genome. That is, except for the DNA of interest, the net effect is that sequences which are not normally present in mycobacteria (i.e., plasmid sequences) are not present in the recombinant mycobacterial cells.

### Overview of uses and advantages of integration vectors of the present invention

There are numerous uses for and advantages of the integration vector of the present invention. Several of these are described below, as is the use of the integration vector in constructing expression or vaccine vehicles. As a result of the present invention, DNA of interest encoding a lymphokine or immunopotentiator is integrated into the mycobacteria genome and expression of the DNA is regulated with a regulated promoter region, with the promise that the promoter region does not comprise the cI promoter.

New genetic approaches to understanding questions of disease pathogenesis are now available. The integration vector, may find and utility in the insertional mutagenesis and marking of genes of pathogenic mycobacteria, by homologous recombination, with the aim of identifying specific genetic functions required for virulence and pathogenesis. For example, using this vector and mycobacteria (e.g., M. smegmatis, M. bovis-BCG), virulence genes of M. tuberculosis or M. leprae can be identified and diagnostics (diagnostic tests) developed. By specifically deleting or replacing those genes, it may be possible to develop a more specific and effective attenuated vaccine against tuberculosis than the current M. bovis-BCG vaccine. Alternatively, as specific protective antigens for tuberculosis and leprosy are identified by study of antigens recognized by T cells from resistant individuals, it will now be possible to introduce and express them in currently existing M. bovis-BCG vaccines.

### Overview of uses and advantages of recombinant mycobacteria

Vehicles the mycobacteria of the present invention can be used, for example, as vaccines

Vaccine vehicles of the present invention can be used to treat human cancers, such as bladder cancers or melanomas. In this context, recombinant mycobacteria which contain and express interferon α, β and/or γ, one or more interleukin (interleukins 1-7) and/or TNF α or β are particularly useful.

It is possible to include in the integration vector DNA encoding a signal sequence and, thus, provide a means by which the expressed immunopotentiator or lymphokine is made in the cytoplasm and then secreted at the cell walls. For example, the signal sequence from α antigen, which is secreted in mycobacteria, could be used. Alternatively, the signal sequence for β-galactosidase, agarase or α amylase could be used.

The present invention will now be illustrated by the following examples, which are not to be considered limiting in any way.

### Example 1 Construction of a recombinant plasmid for introduction of the Kan gene into M. bovis-BCG and of a marked target BCG

The DH5α bacterial strain was used to prepare a recombinant plasmid. (Bethesda Research Lab, Maryland). M. bovis-BCG (Moreau) genomic DNA was prepared by standard procedures.

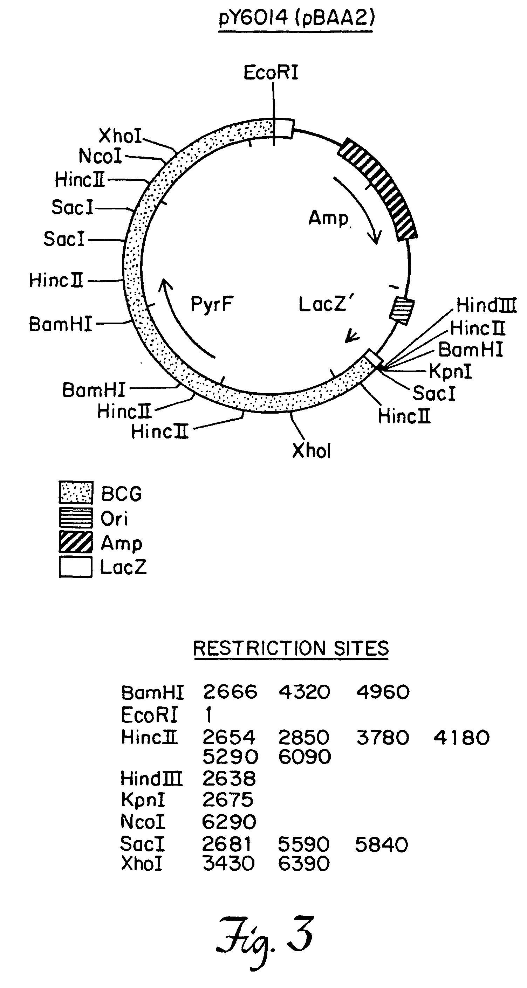

The starting vector pY6014 was produced by inserting a Sac1 partial-EcoRI 4510 bp fragment cloned from pY6013 into pUC19 DNA digested with Sac1 and EcoRI. (See Figure 3) The pY6011 plasmid was obtained by inserting a 5610 bp EcoRI fragment from Y3330 into EcoRI digested pGEM-7Zf⁺ (Promega Biotech). Y3330 is a λgt11 clone from a λgy11 library of BCG DNA, obtained by screening the library with a fragment of the BCG PryF gene. The pY6014 contains the mycobacterial PyrF gene and 1.4-1.6 kb of flanking mycobacterial DNA on either side of the PyrF gene.

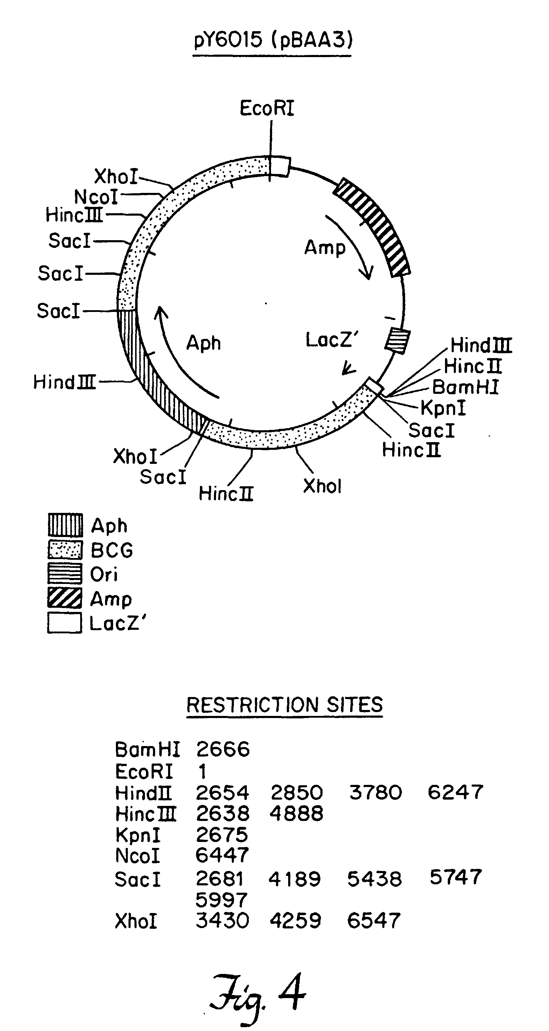

The aph gene which encodes kanamycin resistance was obtained from pY6005 and inserted in the pY6014 recombinant plasmid to replace the PyrF coding sequences, using standard techniques. (pY6005; (Kan^{R}) kanamycin resistance cartridge derived from Tn903 was inserted in pUC4 Sac1, a derivative of pUC4 with pUC1 replacing the Sac1 site in the polylinker). The aph gene was cloned into HincII 6.1 kb fragment from pY6014, as a BamHI filled in fragment from pY6005. This produced the recombinant plasmid pY6015 which was used to produce the marked target BCG strain. (See Figure 4)

### Isolation of marked target BCG containing the Kan gene

As diagrammed in Figure 1, a marked target BCG strain was produced by transforming BCG with the recombinent plasmid pY6015 discribed above. This procedure was performed as follows: BCG cells were grown in suspension culture at optical density (OD) 0.5, concentrated 50 fold in electroporation buffer PSB (phosphate sucrose buffer) and transferred to a curvette. After addition of 10 µg of plasmid DNA, one electrical pulse of 6,250 V/cm and 25 µF was given and the cells were resuspended in 10 ml medium, grown at 37°C for 2 hours and plated.

Marked target BCG were isolated by plating the transformation on Middlebrook 7H10 agar plates containing 20 µg/ml kanamycin and 1 mg/ml FOA.

### Example 2 Construction of an integration vector for introduction of DNA of interest into marked target M. bovis-BCG

An integration vector capable of transforming the marked target BCG (produced in Example 1) was engineered which contained the mycobacterial PyrF gene, DNA sequences homologous to sequences flanking mycobacterial PyrF sequences necessary for homologous recombination, the mycobacterial hsp70 promoter and DNA of interest (i.e., the HIV1 or SIV1 gag, pol or env gene). This procedure is described below and diagrammed in Figure 2.

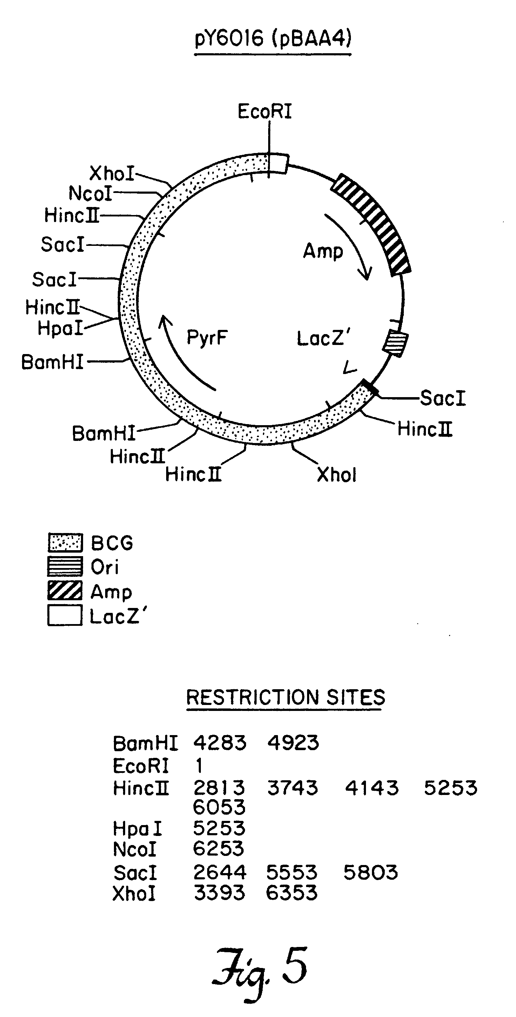

The recombinant vector pY6014, described in Example 1, which contains the mycobacterial PyrF gene and flanking DNA sequences was digested with KpnI and HindIII, blunt ended and self-ligated to produce the vector pY6016. (See Figure 5). This procedure was performed according to standards procedures. This results in the removal of several restriction sites present in pY6014 located in the original polylinker DNA sequence.

The hsp70 M. tuberculosis EcoRI-HindIII 1.8 kb filled in fragment was obtained from Y3334 (an M13 mp18 phage containing the EcoRI - Kpm 1.8 Kb fragment from λgt11 clone Y3111 which was obtained by screening a λgt11 library of M. tuberculosis DNA with antibodies against the 70 kDa antigen), and cloned into the Hpal site of pY6016 (106 bp downstream of PyrF) using standard procedures.

As shown in Figure 6, this results in the recombinant plasmid pY6017, which contains the hsp70 promoter and partial coding sequence as well as mycobacterial (BCG) flanking sequences necessary for double homologous recombination with the marked target BCG produced in Example 1. Using this vector (pY6017), 6 integration vectors were produced having DNA encoding the HIV1 gag, pol or env gene or the SIV1 gag, pol or env ene. Each of these vectors was produced by the following procedure in which the hsp70 coding region was replaced with the DNA of interest.

### Insertion of DNA of interest in pY6017

The NcoI site in the hsp70 insert is at the ATG of hsp70 gene. DNA encoding a gene of interest was cloned into this vector (pY6017) using a polymerase chain reaction (PCR) on the gene of interest as template. The two oligonucleotides used in the reaction contain at the two ends two convenient restriction sites (in this case, NcoI for the oligonucleotides at the 5' end of the gene, and one of the sites of the polylinker for the oligonucleotides at the 3' end of the gene). The PCR product was digested with the two enzymes chosen for the oligonucleotides and gel purified. The.vector was cut to completion with one of the enzymes of the polylinker (KpnI, XbaI) and to partial digestion with Ncol. If NcoI was present in the gene of interest, the NcoI site was blunted prior to digestion with the KpnI or XbaI enzyme. The PCR product was filled in at the 5' end, and cut with the 3' end enzyme.

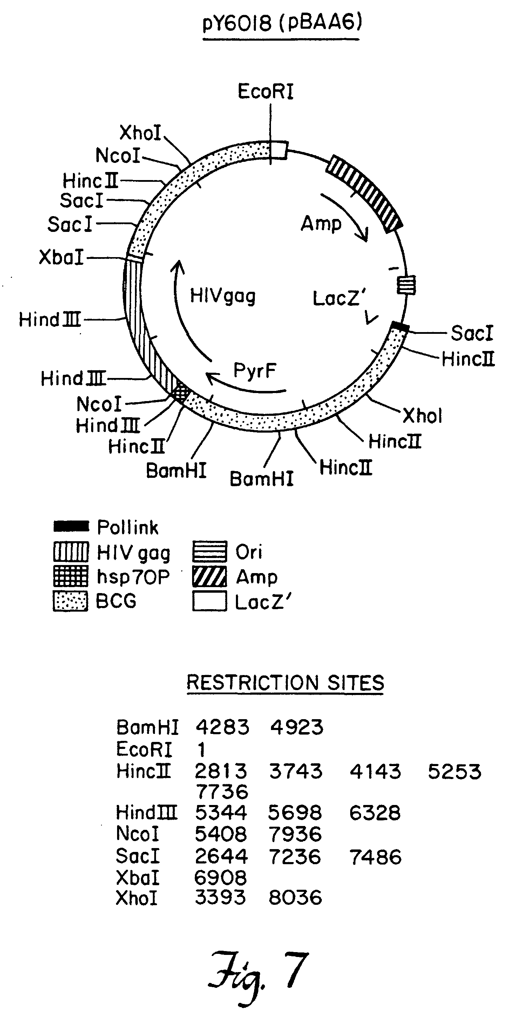

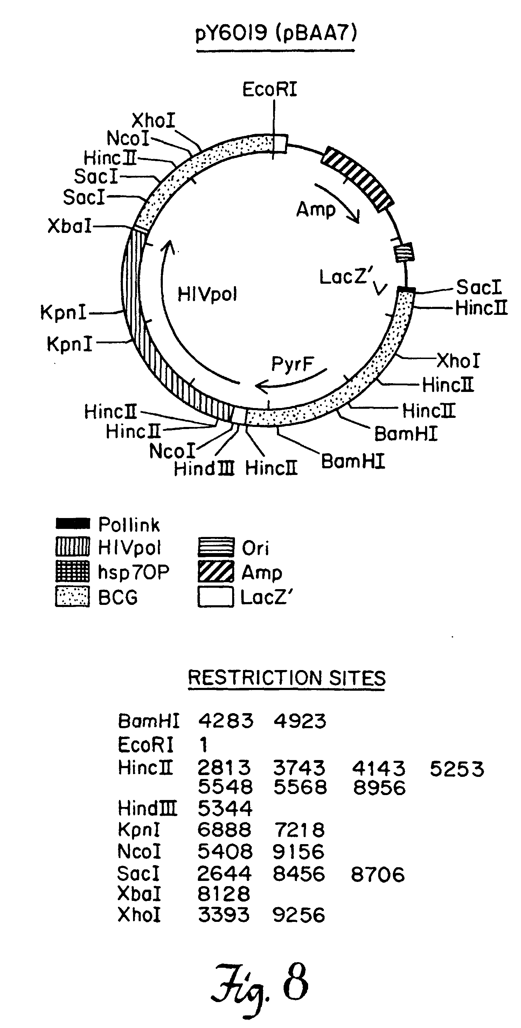

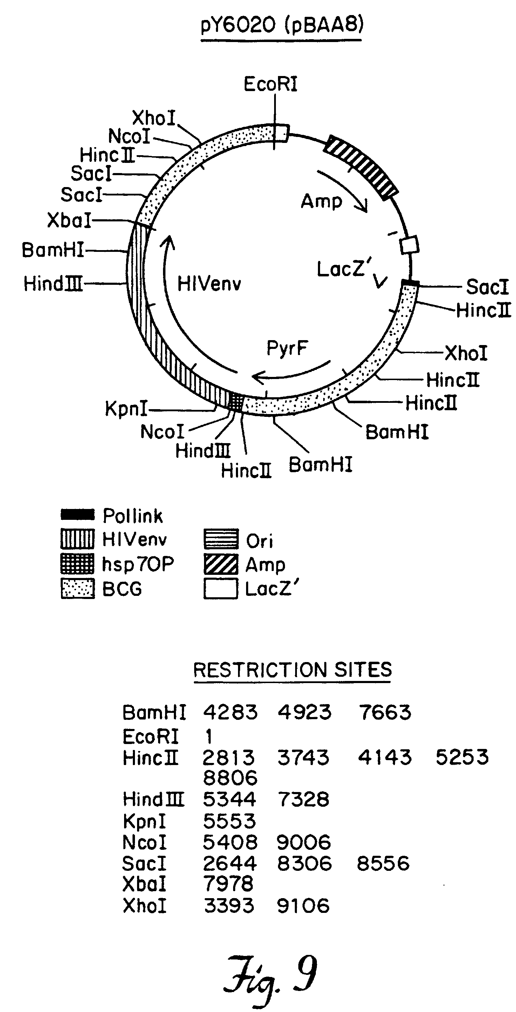

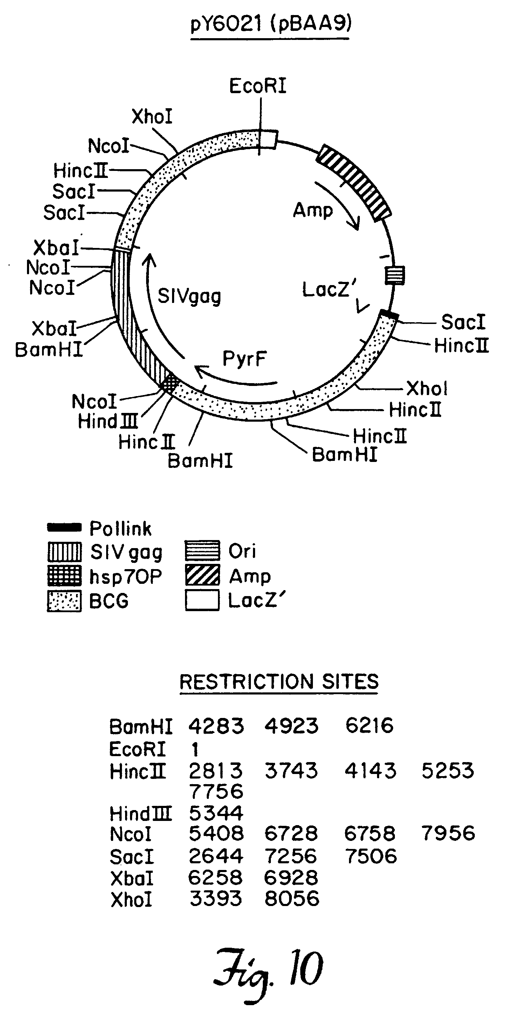

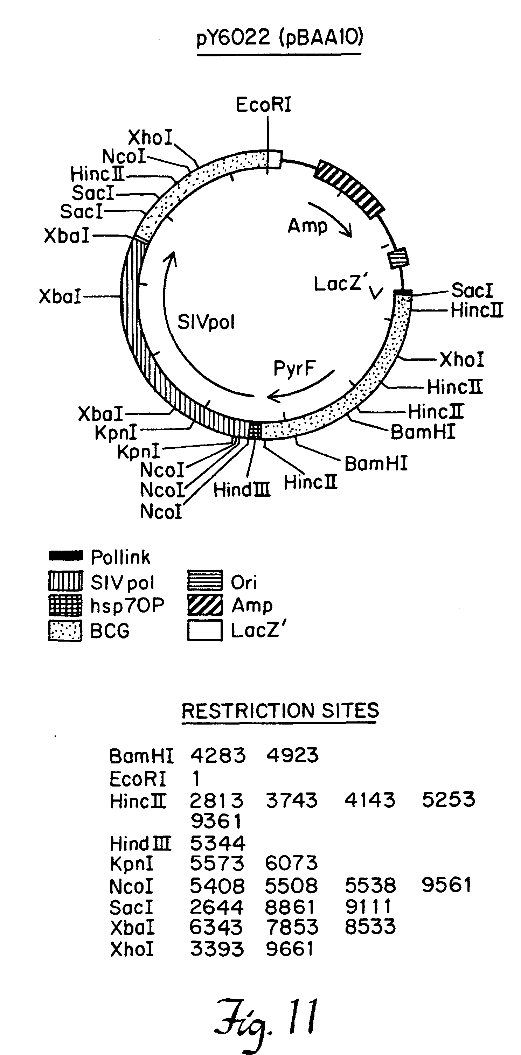

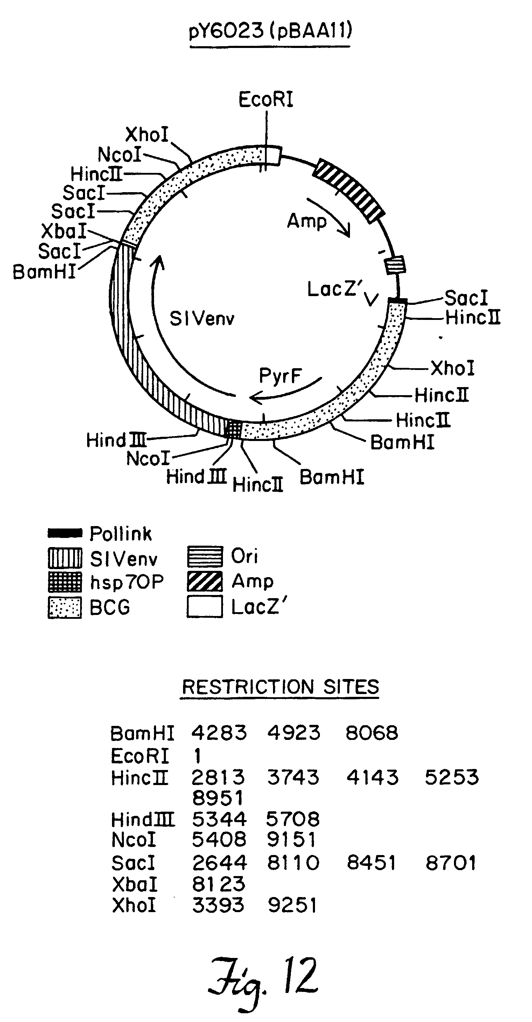

The following recombinant plasmid vectors were produced:
- pY6018:: the HIV1 gag gene was synthesized as a 1500 bp Ncol-Xbal fragment using PCR and cloned into Ncol partial-Xbal digested pY6017 (See Figure 7)
- pY6019:: the HIV1 pol gene was synthesized as a 2720 bp Ncol-Xbal fragment using PCR and was inserted into Ncol partial-Xbal digested pY6017 (See Figure 8)
- pY6020:: the HIV1 env gene was synthesized as a 2570 bp Ncol-Xbal fragment using PCR and was inserted into Ncol partial-Xbal digested pY6017 (See Figure 9)
- pY6021:: the SIV1 gag gene was synthesized as a 1520 bp blunt- Kpnl fragment using PCR and was inserted into Ncol partial-filled in Kpnl digested pY6017 (See Figure 10)
- pY6022:: the SIV1 pol gene was synthesized as a 3125 bp Smal fragment using PCR and was inserted into Ncol partial-Xbal digested and filled in pY6017 (See Figure 11)
- pY6023:: the SIV1 env gene was synthesized as a 2750 bp Smal-Xbal fragment using PCR and was inserted into Ncol partial filled in and Xbal digested pY6017 (See Figure 12)

## Claims

1. A recombinant mycobacterium having heterologous DNA encoding a lymphokine or immunopotentiator stably incorporated into the genomic DNA thereof, expression of the heterologous DNA being under the control of a regulated promoter region, with the proviso that the promoter region does not comprise the cl promoter.

2. The mycobacterium of claim 1 wherein the promoter region comprises a mycobacterial promoter region.

3. The mycobacterium of claim 1 or claim 2 wherein the lymphokine is an interferon or an interleukin.

4. The mycobacterium of claim 3 wherein the interferon is IFN_{γ}.

5. The mycobacterium of claim 3 wherein the interleukin is IL-2.

6. The mycobacterium of any one of the preceding claims wherein the lymphokine or immunopotentiator is secreted.

7. The mycobacterium of claim 6 wherein the heterologous DNA further comprises a portion encoding a signal sequence for secretion.

8. The mycobacterium of claim 7 wherein the signal sequence is from α antigen, β-galactosidase, agarase or a amylase.

9. The mycobacterium of any one of the preceding claims wherein the heterologous DNA is under the control of an expression cassette.

10. The mycobacterium of any one of the preceding claims which is selected from:
(a) *Mycobacterium smegmatis*;
(b) *Mycobacterium bovis-BCG*;
(c) *Mycobacterium avium*;
(d) *Mycobacterium phlei*;
(e) *Mycobacterium fortuitum*;
(f) *Mycobacterium lufu*;
(g) *Mycobacterium paratuberculosis*;
(h) *Mycobacterium habana*;
(i) *Mycobacterium scrofulaceum*;
(j) *Mycobacterium intracellulare*;
(k) generic variants of any of the foregoing.

11. The mycobacterium of any one of the preceding claims wherein the heterologous DNA:
(a) encodes IL-2 or IFN_{*y*}; and
(b) is under the control of a mycobacterial promoter; and
(c) further comprises a signal sequence for secretion of the IL-2 or IFN_{γ}.

12. An immunogenic/pharmaceutical composition (eg a vaccine) comprising the mycobacterium of any one of the preceding claims.

13. An integration vector (e.g. plasmid) for stable integration into the genomic DNA of a mycobacterium, the vector comprising:
(a) DNA sequences homologous to DNA sequences in the mycobacterial genome sufficient to promote double homologous recombination with the mycobacterial genome;
(b) DNA encoding a regulated promoter region, with the proviso that the promoter region does not comprise the cl promoter;
(c) DNA encoding a selectable marker; and
(d) heterologous DNA encoding a lymphokine or immunopotentiator for expression in the mycobacterium.

14. The vector of claim 13 wherein the heterologous DNA is as defined in any one of claims 1-11.

15. The mycobacterium of any one of claims 1-11 or the composition of claim 12 for use in cancer therapy (e.g. for use in the treatment of bladder cancer or melanomas).

## Patentansprüche

1. Rekombinantes Mycobakterium, das eine heterologe DNA aufweist, die ein Lymphokin oder ein die Immunantwort verstärkendes Mittel kodiert, stabil eingebaut in deren genomische DNA, wobei die Expression der heterologen DNA unter der Kontrolle einer regulierten Promotor-Region erfolgt, unter dem Vorbehalt, dass die Promotor-Region den cl-Promotor nicht umfasst.

2. Mycobakterium nach Anspruch 1, wobei die Promotor-Region eine mycobakterielle Promotorregion umfasst.

3. Mycobakterium nach Anspruch 1 oder Anspruch 2, wobei das Lymphokin ein Interferon oder ein Interleukin ist.

4. Mycobakterium nach Anspruch 3, wobei das Interferon IFN_{γ} ist.

5. Mycobakterium nach Anspruch 3, wobei das Interleukin IL-2 ist.

6. Mycobakterium nach einem der vorhergehenden Ansprüche, wobei das Lymphokin oder das die Immunantwort verstärkende Mittel sezerniert wird.

7. Mycobakterium nach Anspruch 6, wobei die heterologe DNA weiterhin einen Anteil umfasst, der eine Signalsequenz zur Sekretion kodiert

8. Mycobakterium nach Anspruch 7, wobei die Signalsequenz aus einem α-Antigen, β-Galaktosidase, Agarase oder α-Amylase stammt.

9. Mycobakterium nach einem der vorhergehenden Ansprüche, wobei die heterologe DNA unter der Kontrolle einer Expressionskassette steht.

10. Mycobakterium nach einem der vorhergehenden Ansprüche, das aus folgendem ausgewählt ist:
(a) Mycobakterium smegmatis;
(b) Mycobakterium bovis-BCG;
(c) Mycobakterium avium;
(d) Mycobakterium phlei;
(e) Mycobakterium fortuitum;
(f) Mycobakterium lufu;
(g) Mycobakterium paratuberculosis;
(h) Mycobakterium habana;
(i) Mycobakterium scrofillaceum;
(j) Mycobakterium intracellulare;
(k) generische Varianten irgendeines des Vorhergehenden.

11. Mycobakterium nach einem der vorhergehenden Ansprüche, wobei die heterologe DNA:
(a) IL-2 oder IFN_{γ} kodiert; und
(b) unter der Kontrolle eines mykobakteriellen Promotors steht; und
(c) weiterhin eine Signalsequenz zur Sekretion des IL-2 oder IFN_{γ} umfosst.

12. Immunogene/pharmazeutische Zusammensetzung (beispielsweise Vakzine), die das Mycobakterium nach einem der vorhergehenden Ansprüche umfasst.

13. Integrationsvektor (beispielsweise Plasmid) zur stabilen Integration in die genomische DNA eines Mykobakteriums, wobei der Vektor folgendes umfasst:
(a) DNA-Sequenzen, die zu DNA-Sequenzen im mykobakteriellen Genom ausreichend homolog sind, um eine doppelt homologe Rekombination mit dem mykobakteriellen Genom zu fördern;
(b) eine regulierte Promotor-Region kodiert, unter dem Vorbehalt, dass die Promotor-Region den cl-Promotor nicht umfasst;
(c) DNA, die einen selektierbaren Marker kodiert; und
(d) heterologe DNA, die ein Lymphokin oder ein die Immunantwort verstärkendes Mittel zur Expression im Mykobakterium kodiert.

14. Vektor nach Anspruch 13, wobei die heterologe DNA wie in einem der Ansprüche 1-11 definiert ist.

15. Mykobakterium nach einem der Ansprüche 1-11 oder Zusammensetzung nach Anspruch 12 zur Verwendung in der Krebstherapie (beispielsweise zur Verwendung in der Behandlung eines Blasenkarzinoms oder von Melanomen).

## Revendications

1. Une mycobactérie recombinante possédant un ADN hétérologue codant pour une lymphokine ou un immunostimulant incorporé de manière stable dans l'ADN génomique de celle-ci, l'expression de l'ADN hétérologue étant sous le contrôle d'une région promotrice régulée, à la condition que la région promotrice ne comprenne pas le promoteur cl.

2. Mycobactérie selon la revendication 1 dans laquelle la région promotrice comprend une région promotrice mycobactérienne.

3. Mycobactérie selon la revendication 1 ou 2 dans laquelle la lymphokine est un interféron ou une interleukine.

4. Mycobactérie selon la revendication 3 dans laquelle l'interféron est l'IFN γ

5. Mycobactérie selon la revendication 3 dans laquelle l'interleukine est l'IL-2.

6. Mycobactérie selon l'une quelconque des revendications précédentes dans laquelle une lymphokine ou un immunostimulant est sécrété.

7. Mycobactérie selon la revendication 6 dans laquelle l'ADN hétérologue comprend en outre une partie codant une séquence signal pour la sécrétion.

8. Mycobactérie selon la revendication 7 dans laquelle la séquence signal est issue d'un antigène α, β-galactosidase, agarase, ou α-amylase.

9. Mycobactérie selon l'une quelconque des revendications précédentes dans laquelle l'ADN hétérologue est sous le contrôle d'une cassette d'expression.

10. Mycobactérie selon l'une quelconque des revendications précédentes choisie parmi :
*(a) Mycobactérium smegmatis*
*(b) Mycobactérium bovis-BCG*
*(c) Mycobactérium avium*
*(d) Mycobactérium phlei*
*(e) Mycobaclérium fortuitum*
*(f) Mycobactérium lufu*
*(g) Mycobactérium paratuberculosis*
*(h) Mycobactérium habana*
*(i) Mycobactérium scrofulaceum*
(j) *Mycobactérium intracellulare*
(k) des variants génériques des mycobactéries précédentes

11. Mycobactérie selon l'une quelconque des revendications précédentes dans laquelle l'ADN hétérologue :
(a) code pour IL-2 ou IFNγ ; et
(b) est sous le contrôle d'un promoteur de mycobactérie ; et
(c ) comprend en outre une séquence signal pour la sécrétion de l'IL-2 ou de l'IFNγ.

12. Une composition immunogène/pharmaceutique (par exemple un vaccin) comprenant la mycobactérie selon l'une quelconque des revendications précédentes.

13. Un vecteur d'intégration (par exemple un plasmide) pour une intégration stable dans l'ADN génomique d'une mycobactérie, le vecteur comprenant :
(a) des séquences d'ADN homologues à des séquences d'ADN dans le génome de la mycobactérie suffisantes pour favoriser une double recombinaison homologue avec le génome de la mycobactérie ;
(b) un ADN codant pour une région promotrice régulée, à la condition que la région promotrice ne comprenne pas le promoteur cl ;
(c) un ADN codant pour un marqueur sélectionnable ; et
(d) un ADN hétérologue codant pour une lymphokine ou un immunostimulant pour l'expression dans la mycobactérie.

14. Le vecteur selon la revendication 13 dans lequel l'ADN hétérologue est tel que défini dans l'une quelconque des revendications 1-11.

15. Mycobactérie selon l'une quelconque des revendications 1-11 ou la composition de la revendication 12 pour utilisation dans la thérapie contre le cancer (par exemple pour une utilisation dans le traitement du cancer de la vésicule ou de mélanomes).
